# EUROPÄISCHE PATENTSCHRIFT

(11) **EP 1 847 269 B1**
(45) Veröffentlichungstag und Bekanntmachung des Hinweises auf die Patenterteilung: **30.06.2010**
(21) Anmeldenummer: 07007280.6
(22) Anmeldetag: 07.04.2007
(51) Int. Cl.: A61K 31/621, A61L 33/04, A61M 1/16, A61M 1/36, A61P 7/04

(54) **Verwendung von Acetylsalicylsäure (ASS) beim Einsatz einer Membranlunge**
Utilisation of acetyl salicylic acid (ASA) when using a membrane lung
Emploi d'acides d'acétylsalicyle (ASS) lors de l'utilisation d'un poumon à membrane

(30) Priorität: 21.04.2006 DE 102006020492
(43) Veröffentlichungstag der Anmeldung: 24.10.2007
(73) Patentinhaber: NovaLung GmbH, 72379 Hechingen (DE)
(72) Erfinder: Philipp, Alois, 93080, Pentling (DE); Schmid, Franz-Xaver, 93309, Kelheim (DE); Birnbaum, Dietrich, 93053, Regensburg (DE)
(74) Vertreter: Graf von Stosch, Andreas

(56) Entgegenhaltungen:
- EP-A1- 0 665 023
- WO-A-00/01432
- GLAUBER, M; SZEFNER, J; SENNI, M; GAMBA, A; MAMPRIN, F; FIOCCHI, R; SOMASCHINI, M; FERRAZZI, P.: "Reduction of haemorraghic complication during mechanically assisted circulation with the use of a multi-system anticoagulation protocol" THE INTERNATIONAL JOURNAL OF ARTIFICIAL ORGANS, Bd. 18, Nr. 10, Oktober 1995 (1995-10), Seiten 649-655, XP009087534
- ROSE, E.A; LEVIN, H.R; MEHMET, C.OZ; FRAZIER, H; MACMANUS, Q; BURTON, N,A; LEFRAK, E,A: "Artificial circulatory support with textured interior surfaces" CIRCULATION, Bd. 90, Nr. 5, November 2004 (2004-11), Seiten 87-91, XP009087605
- TABUCHI ET AL: "Hemostatic function of aspirin-treated platelets vulnerable to cardiopulmonary bypass" JOURNAL OF THORACIC AND CARDIOVASCULAR SURGERY, MOSBY-YEAR BOOK, INC., ST. LOUIS, MO, US, Bd. 110, Nr. 3, September 1995 (1995-09), Seiten 813-818, XP005143388 ISSN: 0022-5223
- TOWNSEND E R ET AL: "Preservation of platelets during extracorporeal circulation in sheep. A comparison between aspirin and sulfinpyrazone." CIRCULATION RESEARCH AUG 1981, Bd. 49, Nr. 2, August 1981 (1981-08), Seiten 452-457, XP002445137 ISSN: 0009-7330
- FISCHER ET AL: "Bridge to lung transplantation with the novel pumpless interventional lung assist device NovaLung" JOURNAL OF THORACIC AND CARDIOVASCULAR SURGERY, MOSBY-YEAR BOOK, INC., ST. LOUIS, MO, US, Bd. 131, Nr. 3, März 2006 (2006-03), Seiten 719-723, XP005329170 ISSN: 0022-5223

## Beschreibung

Die vorliegende Erfindung betrifft die Herstellung eines Medikamentes, das einem human Patienten im Zusammenhang mit einer Behandlung des Patienten mit einer Membranlunge verabreicht wird, um eine Ablagerung von Blutbestandteilen in der Membranlunge zu vermeiden oder zu verringern.

Ein extrakorporales Lungenunterstützungssystem, das auf einer Membranlunge beruht, wird von der Anmelderin unter der Bezeichnung NovaLung iLA (interventional Lung Assist) vertrieben.

Blutseitig wird das NovaLung iLA unmittelbar an den Blutkreislauf eines Patienten über perkutane arterielle und venöse Kanulierung angeschlossen. Mit dem NovaLung iLA ist es ohne Verwendung einer blutseitigen Pumpe möglich, das von dem Herzen des Patienten pulsatil durch die Membranlunge gepumpte Blut von Kohlendioxid zu befreien und unter den Limitationen des arteriellen Bluteinstroms zu oxigenieren.

Die Bedeutung des NovaLung iLA und allgemein von Membranlungen und künstlichen Lungensystemen ist vor dem Hintergrund zu sehen, dass Lungenerkrankungen nach den Statistiken der Weltgesundheitsorganisation die dritthäufigste Todesursache darstellen. Bei einem Lungenversagen besteht derzeit nur die Möglichkeit der maschinellen Beatmung, wobei es sich jedoch nicht um eine Lungenunterstützung handelt, denn die kranke Lunge wird nicht therapiert, vielmehr wird lediglich der lebensnotwendige Gasaustausch sichergestellt.

Eine mechanische Beatmung vermag zwar den Gasaustausch in fast allen Patienten aufrechtzuerhalten, erzeugt aber durch den unnatürlichen positiven Atemwegsdruck eine Schädigung der Lunge und anderer Organe, die als Ventilator Associated Lung Injury (VALI) bekannt ist. Mit dem NovaLung iLA ist es erstmals möglich, durch eine Ventilation außerhalb der Lunge eine sehr protektive Beatmung oder z.B. eine Überbrückung zur Lungentransplantation zu erzielen und den VALI zu vermeiden.

Die Lungentransplantation stellt jedoch ein medizinisch extrem aufwändiges Verfahren dar, das zudem mit einem hohen Risiko für den Patienten verbunden ist. Abgesehen davon, dass dieses Therapiekonzept nur Patienten vorbehalten ist, die eine isolierte Lungenerkrankung haben und anderweitig gesund sind, sind die Langzeitergebnisse nicht zufriedenstellend. Hinzu kommt, dass auf Grund der geringen Menge der zur Verfügung stehenden Spenderorgane weltweit jährlich nur eine geringe Anzahl von Lungentransplantationen durchgeführt werden kann, die dem tatsächlichen Bedarf nicht genügt.

Vor diesem Hintergrund besteht ein signifikanter Bedarf an einem Lungenersatzverfahren als lebensverlängernde Behandlung ("Destination Therapy"). Ein extrakorporales oder implantierbares Lungenunterstützungssystem, das sowohl das Blut oxygenieren als auch Kohlendioxid aus dem Blut entfernen kann, ist also für Patienten von allerhöchstem Wert. Derartige Lungenunterstützungssysteme können jedoch nicht nur bei Patienten, die nicht für eine Transplantation in Frage kommen, sondern auch bei Patienten angewendet werden, die auf eine Lungentransplantation warten, aber während der Wartezeit ein endgradiges Lungenversagen entwickeln, das zur Beatmung zwingt.

Dass künstliche Lungenunterstützungssysteme wie das NovaLung iLA hierfür prinzipiell einsetzbar sind, konnte in einer klinischen Studie gezeigt werden; siehe Fischer et al.: BRIDGE TO LUNG TRANSPLANTATION WITH THE NOVEL PUMPLESS INTERVENTIONAL LUNG ASSIST DEVICE NOVALUNG, in J Thorac Cardiovasc Surg. 2006;131(3):719-23.

Im Rahmen dieser Studie konnten Patienten, die trotz maschineller Beatmung ein beatmungsrefraktäres Lungenversagen entwickelten, mittels des Lungenunterstützungssystems NovaLung iLA erfolgreich zur Transplantation überbrückt werden.

Derzeit können solche Systeme auf der Intensivstation für einen Zeitraum von wenigen Wochen eingesetzt werden, weil ein längerer Einsatz der NovaLung iLA durch eine Neointimabildung und andere Faktoren nicht möglich ist, außer man nimmt regelmäßig einen Austausch der Membranlunge vor.

Negative Begleiterscheinungen sind auch bei dem anderweitigen klinischen Einsatz von organunterstützenden Systemen mit Fremdoberflächen bekannt, die mit Blut in Berührung kommen. Beispiele für derartige organunterstützende Systeme sind Herz-Lungen-Maschinen mit Oxygenator, mechanische Blutpumpen, Hämodialyse und Herzunterstützungssysteme, wie bspw. Kunstherzen.

Der Kontakt des Blutes mit den unphysiologischen Fremdoberflächen führt dabei zur Aktivierung des Koagulations-, Komplement- und Fibrinolysesystems sowie weiterer zellulärer Blutbestandteile, was zu einer Thrombenbildung an den Fremdoberflächen und damit zu einem Zusetzen bspw. der in dem Oxygenator verwendeten Gasaustauschmembran führt. Diese Effekte führen schon in kurzer zeit zu einem merklichen Nachlassen der Gasaustauschleistung und einer merklichen Zunahme des Strömungswiderstandes der Membranlunge für den vom Herzen des Patienten durch die Membranlunge getriebenen Blutstromes, so dass schon nach kurzer Standzeit von wenigen Tagen die Membranlunge ausgetauscht werden muss.

Für Herz-Lungen-Maschinen ist es bekannt, der Thrombenbildung durch eine Antikoagulation vorzubeugen, die durch die Gabe von Heparin induziert wird. Als alternative Antikoagulantien können Hirudin, Danaparoid-Natrium, Ancrod oder Argatroban eingesetzt werden, die jedoch alle zu schwerwiegenden Nebenwirkungen führen können; siehe die Inauguraldissertation von Schenck zu Schweinsberg: Management der Antikoagulation während des kardiopulmonalen Bypasses bei Patienten mit einer Heparin-induzierten Thrombozytopenie, Gießen 2004.

Glauber et al.: Reduction of haemorrhagic complications during mechanically assisted circulation with the use of a multi-system anticoagulation protocol, in Int. J. of Artificial Organs 1995, 18: 649-655, beschreiben eine Multi-Medikamenten-Therapie, um den kontinuierlichen Angriff von Fremdoberflächen auf das Koagulationssystem zu dämpfen. Sie schlagen dafür die Verabreichung von Dipyridamol zusammen mit Heparin, Aprotinin und Aspirin vor.

Rose et al.: Artificial Circulatory Support With Textured Interior Surfaces, in Circulation, Band 90, Nr. 5, November 2004 (2004-11, Seiten 87 bis 91) beschreiben ein Kreislaufunterstützungsgerät mit angerauten Innenoberflächen, wobei verschiedene antikoagulative Behandlungen, einschließlich Aspirin, erwähnt werden.

Townsend et al.: "Preservation of Platelets during Extracorporeal Circulation in Sheep" in Circulation Research, August 1981, Band 49, Nr. 2, August 1981 (1981-08), Seiten 452 bis 457, beschreiben Experimente an Schafen, bei denen im Zusammenhang mit dem Einsatz eines Oxygenators antikoagulative Substanzen, beispielsweise Aspirin, eingesetzt wurden.

Zur Vermeidung von Thromboembolien bei mechanischen Herzunterstützungssystemen schlagen Etz et al.: "Analysis of platelet function during left ventricular support with the Incor and Excor system", in Heart Surg Forum, 2004; 7(5):E423-7, vor, ein Antikoagulationsprotokoll mit Heparin, Aspirin und Clopidogrel zu verwenden.

Bhatt et al.: "Clopidogrel and Aspirin versus Aspirin alone for the Prevention of Atherothrombic Events", in N Engl J Med 2006, beschreiben, dass Aspirin in täglichen Dosen von 75 bis 162 mg die Herzinfarkt- und Schlaganfallgefahr reduziert. Dalen, "Aspirin to prevent heart attack and stroke: what's the right dose", in Am J Med, 2006; 119(3):198-202, beschreibt, dass eine tägliche Dosis von 160 mg Aspirin geeignet ist, das Risiko für Herzinfarkt und Schlaganfall zu vermindern, erwähnt aber auch, dass bei diesen Dosen ebenso wie bei 80 mg Aspirin täglich die Gefahr "schwerer Blutungen" besteht, also Blutungen mit hämodynamischer Beeinträchtigung und Transfusionsbedarf.

Die WO 00/01432 beschreibt einen Oxygenator mit Oberflächenbeschichtung, wobei die Oberflächenbeschichtung ein antikoagulatives Agens, beispielsweise Aspirin, enthalten kann.

Die EP 0 665 023 B1 beschreibt ein medizinisches Material, das ein Antiplättchenmittel wie bspw. Aspirin enthält. Aus diesem Material sollen Fremdoberflächen von Organersatzsystemen gefertigt werden, um der Thrombogenese entgegenzuwirken. Beim Kontakt mit Blut soll das Aspirin kontinuierlich und allmählich freigesetzt werden, so dass es im Blutstrom eine Konzentration von 4ug/ml Blut aufweist.

Vor diesem Hintergrund ist es Aufgabe der vorliegenden Erfindung, ein Medikament der eingangs genannten Art bereitzustellen, das unter größtmöglicher Vermeidung von Nebenwirkungen den Einsatz von Membranlungen nicht nur zur kurzzeitigen, sondern auch zur mittelfristigen und langfristigen Lungenunterstützung erlaubt sowie den Einsatz eines auf einer Membranlunge beruhenden, implantierbaren Lungenunterstützungssystems ermöglicht.

Erfindungsgemäß wird diese Aufgabe dadurch gelöst, dass bei der eingangs erwähnten Herstellung Acetylsalicylsäure (ASS) verwendet wird, und dass das Verabreichen des Medikamentes es umfasst, dass ASS anfänglich täglich einmal, mehrmals oder kontinuierlich in einer therapeutisch wirksamen täglichen Menge verabreicht wird, die unterhalb des Risikobereiches für analgetische Wirksamkeit von ASS liegt.

Die der Erfindung zugrundeliegende Aufgabe wird auf diese Weise vollkommen gelöst.

Die Erfinder der vorliegenden Anmeldung haben nämlich erkannt, dass bei einer Verabreichung auch geringer täglicher Mengen an ASS die Ablagerung von Blutbestandteilen auf den Gasaustauschmembranen von Membranlungen deutlich reduziert bis vollständig vermieden wird. Dass dies durch die alleinige Gabe geringer täglicher Dosen von ASS möglich ist, war ausgehend von dem eingangs diskutierten Veröffentlichungen unerwartet und überraschend.

Bei der Herstellung des Medikamentes wird also Aspirin als alleiniger therapeutischer Wirkstoff verwendet.

Dass in diesem Zusammenhang auf die Gabe von Heparin verzichtet werden kann, hat den weiteren Vorteil, dass eine Heparin induzierte Thrombopenie (HIT) vermieden wird, an der viele Patienten auf Intensivstationen leiden.

Die Erfindung beruht also auf der Verwendung von ASS, um bei einem human Patienten im Zusammenhang mit einer Behandlung des Patienten mit einer Membranlunge eine Ablagerung von Blutbestandteilen in der Membranlunge zu vermeiden oder zu verringern, wobei ASS anfänglich täglich einmal, mehrmals oder kontinuierlich in einer therapeutisch wirksamen täglichen Menge verabreicht wird, die unterhalb des Risikobereiches für analgetische Wirksamkeit von ASS liegt.

Die tägliche Dosis an ASS liegt dabei erfindungsgemäß unterhalb von 500 mg, vorzugsweise unterhalb von 100 mg, weiter vorzugsweise im Bereich von ca. 40 bis ca. 60 mg, sie beträgt bevorzugt ca. 50 mg.

ASS wird dabei entweder in diesen Dosen oral verabreicht, oder es wird eine jeweils entsprechende Äquivalentdosis intravenös appliziert.

Die tägliche Menge an zu verabreichendem ASS ist damit so niedrig, dass bekannten Nebenwirkungen von ASS, also insbesondere schwere Blutungen, also Blutungen mit hämodynamischer Beeinträchtigung und Transfusionsbedarf, zu vernachlässigen sind.

Damit wird durch die erfindungsgemäße Medikamentierung erstmals ein Langzeiteinsatz von extrakorporalen oder implantierbaren Lungenunterstützungssystemen möglich, auf den bisher in kurzen Zeitabständen erforderlichen Austausch einer im Einsatz am Patienten befindlichen Membranlunge kann folglich verzichtet werden.

Damit werden extrakorporale und vor allem auch implantierbare Lungenunterstützungssysteme nicht nur finanziell attraktiver, sie sind insbesondere was eine Implantation angeht, überhaupt erst medizinisch und ethisch vertretbar.

Weitere Vorteile ergeben sich aus der Beschreibung.

Es versteht sich, dass die vorstehend genannten und die nachstehend noch zu erläuternden Merkmale nicht nur in den jeweils angegebenen Kombinationen, sondern auch in anderen Kombinationen oder in Alleinstellung verwendbar sind, ohne den Rahmen der vorliegenden Erfindung zu verlassen.

Ein Ausführungsbeispiel der Erfindung wird in der nachfolgenden Beschreibung unter Bezug auf die beigefügte Zeichnung näher erläutert. Es zeigen:
- Fig. 1: eine rasterelektronenmikroskopische Aufnahme einer Gasaustauchmembran aus einer Membranlunge nach 14-tägigem Einsatz an einem Patienten bei Verabreichung von täglich 50 mg ASS oral; und
- Fig. 2: eine Aufnahme wie in Fig. 1, jedoch ohne Verabreichung von ASS; und
- Fig. 3: die Hemmung der Thrombozytenaggregation bei einem Patienten, dem im Zusammenhang mit der Behandlung mit dem NovaLung iLA täglich 50mg ASS verabreicht wurden.

Einem Patienten, der einer Behandlung mit dem NovaLung iLA bedurfte, wurde 1 Woche vor dem Einsatz der Membranlunge täglich ASS in einer Menge von 50 mg verabreicht. Diese Gabe von 50 mg ASS täglich wurde während des Einsatzes der Membranlunge zunächst beibehalten, eine Woche vor dem Ausbau der Membranlunge wurde das Medikament jedoch abgesetzt. Die Membranlunge NovaLung iLA selbst wurde dabei so verwendet, wie dies von Fischer et al., a.a.O., beschrieben wurde.

Die Membranlunge konnte auf diese Weise für 2 Wochen am Patienten belassen werden, ohne dass es zu einem merklichen Nachlassen der Gasaustauschleistung oder einer merklichen Zunahme des Strömungswiderstandes der Membranlunge für den vom Herzen des Patienten durch die Membranlunge getriebenen Blutstromes kam.

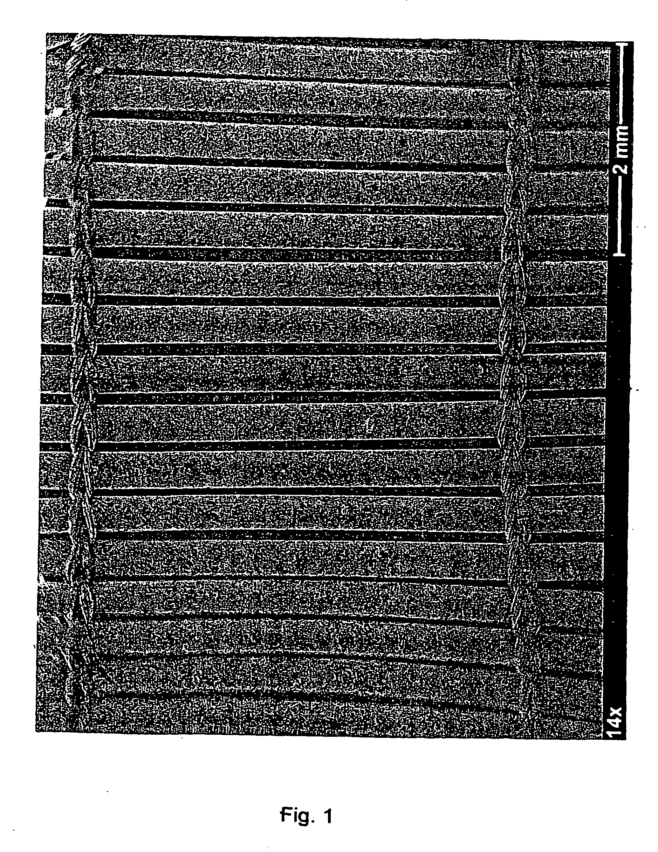

In Fig. 1 ist eine rasterelektronenmikroskopische Aufnahme der Gasaustauchmembran aus der Membranlunge gezeigt, die nach 14-tägigem Einsatz bei dem so behandelten und medikamentierten Patienten wieder ausgebaut und zerlegt wurde. Es ist zu erkennen, dass die Gasaustauschmembran keine Ablagerung von Blutbestandteilen zeigt, sie hätte also deutlich länger im Einsatz bleiben können.

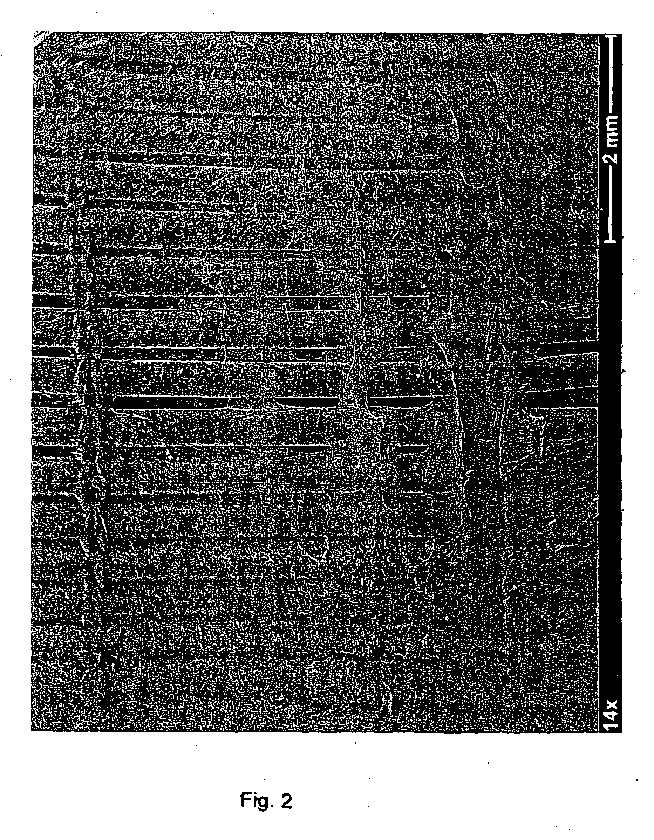

In Fig. 2 ist eine vergleichbare Aufnahme wie in Fig. 1 gezeigt. Der Patient wurde ebenfalls 14 Tage mit der Membranlunge behandelt, allerdings nicht mit ASS medikamentiert. Es ist zu erkennen, dass die Gasaustauschmembran starke Ablagerungen von Blutbestandteilen aufweist, weshalb sie nicht länger hätte eingesetzt werden können, ohne deutliche Leistungsverluste und eine Gefährdung für den Patienten in Kauf zu nehmen.

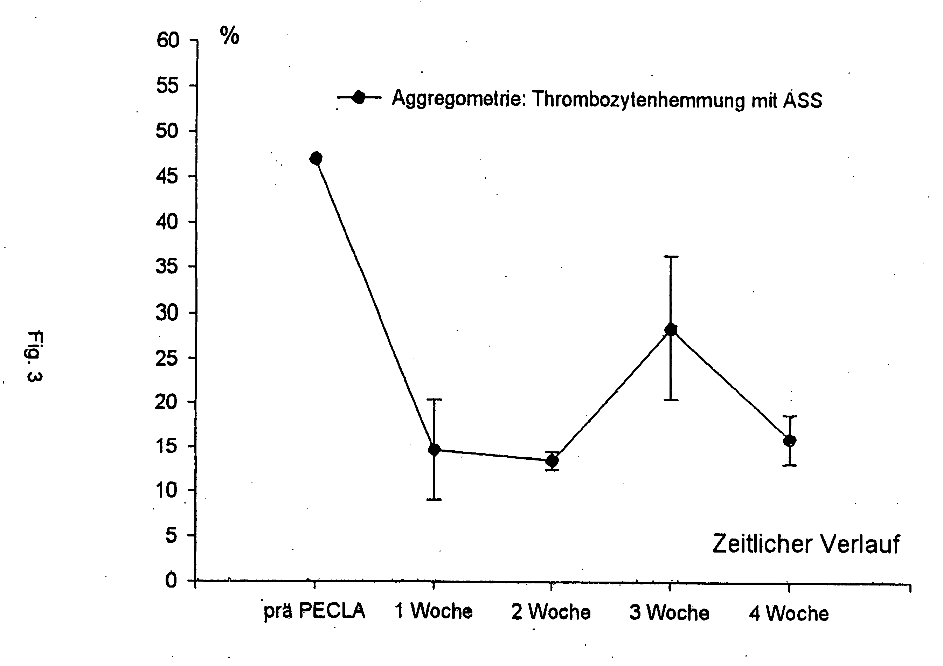

Die Wirkung des ASS-Gabe auf die Standzeit der Membranlunge am Patienten ist nach Erkenntnis der Erfinder der vorliegenden Anmeldungen auch auf die durch ASS bewirkte Verringerung der Thrombozytenaggregation zurückzuführen, wie sie in Fig. 3 für den wie oben behandelten Patienten gezeigt ist.

Die Thrombozytenaggregation wurde dabei nach klinischen Standardverfahren bestimmt.

Die Kurve in Fig. 3 zeigt, dass innerhalb der ersten Woche der ASS Verabreichung die Thrombozytenaggregation auf weit unter 20& abgesenkt war, als nach 1 Woche die Membranlunge angeschlossen wurde. Nach einer weiteren Woche wurde ASS wieder abgesetzt, die Membranlunge aber erst nach noch einer weiteren Woche wieder ausgebaut.

Während der zweiten Woche der ASS Gabe blieb die Thrombozytenaggregation auf ihrem abgesenkten Wert, sie stieg auch nach dem Absetzen des Medikamentes nur allmählich wieder an. 2 Wochen nach dem Beginn der Behandlung mit der Membranlunge, also 1 Woche nach dem Absetzen des Medikamentes, lag die Thrombozytenaggregation immer noch unter 30%.

Diese Ergebnisse belegen insgesamt, dass eine Verabreichung von ASS in einer täglichen Menge, die weit unterhalb der analgetischen Wirksamkeitsmenge von 500 mg und sogar unterhalb der zur Vermeidung von Herzinfarkten und Schlaganfällen empfohlen Wirksamkeitsmenge von über 100 mg liegt, einen Langzeiteinsatz von Membranlungen vermöglicht. Wegen der geringen täglichen ASS Menge sind die bekannten Nebenwirkungen von ASS, also insbesondere schwere Blutungen, also Blutungen mit hämodynamischer Beeinträchtigung und Transfusionsbedarf, zu vernachlässigen.

Wenn ASS täglich einmal, mehrmals oder kontinuierlich in einer täglichen Menge von 40 bis 60 mg, vorzugsweise 50 mg verabreicht wird, kann somit für lange Zeiträume auf den Austausch einer im Einsatz am Patienten befindlichen Membranlunge verzichtet werden.

## Patentansprüche

1. Verwendung von Acetylsalicylsäure (ASS) bei der Herstellung eines Medikamentes, das einem human Patienten im Zusammenhang mit einer Behandlung des Patienten mit einer Membranlunge verabreicht wird, um eine Ablagerung von Blutbestandteilen in der Membranlunge zu vermeiden oder zu verringern, **dadurch gekennzeichnet, dass** das Verabreichen des Medikamentes es umfasst, dass ASS anfänglich täglich einmal, mehrmals oder kontinuierlich in einer therapeutisch wirksamen täglichen Menge verabreicht wird, die unterhalb von 500 mg, vorzugsweise unterhalb von 100 mg liegt, und dass bei der Herstellung des Medikamentes Aspirin (ASS) als alleiniger therapeutischer Wirkstoff verwendet wird.

2. Verwendung nach Anspruch 1, **dadurch gekennzeichnet, dass** die täglich verabreichte Menge an ASS im Bereich von ca. 40 bis ca. 60 mg liegt, vorzugsweise ca. 50 mg beträgt.

3. Verwendung nach Anspruch 1 oder 2, **dadurch gekennzeichnet, dass** die Verabreichung des Medikamentes zumindest 1 Tag, vorzugsweise 5 bis 7 Tage vor Einsatz der Membranlunge beginnt.

4. Verwendung nach einem der Ansprüche 1 bis 3, **dadurch gekennzeichnet, dass** die Verabreichung des Medikamentes zumindest 1 Tag, vorzugsweise 5 bis 7 Tage vor Ende des Einsatzes der Membranlunge beendet wird.

5. Verwendung nach einem der Ansprüche 1 bis 4, **dadurch gekennzeichnet, dass** die Verabreichung des Medikamentes im Zusammenhang mit einer Behandlung mit einem extrakorporalen oder implantierbaren Lungenunterstützungssystem erfolgt.

6. Verwendung nach einem der Ansprüche 1 bis 5, **dadurch gekennzeichnet, dass** die Verabreichung des Medikamentes oral oder intravenös in einer zu der oral verabreichten Menge äquivalenten Dosis erfolgt.

7. Acetylsalicylsäure (ASS) zur Verwendung im Zusammenhang mit einer Behandlung eines humanen Patienten mit einer Membranlunge, um eine Ablagerung von Blutbestandteilen in der Membranlunge zu vermeiden oder zu verringern, **dadurch gekennzeichnet, dass** ASS als Medikament an den Patienten verabreicht wird und das Verabreichen des Medikamentes es umfasst, dass ASS anfänglich täglich einmal, mehrmals oder kontinuierlich in einer therapeutisch wirksamen täglichen Menge verabreicht wird, die unterhalb von 500 mg, vorzugsweise unterhalb von 100 mg liegt, und dass Aspirin (ASS) als alleiniger therapeutischer Wirkstoff verwendet wird.

8. Acetylsalicylsäure (ASS) zur Verwendung nach Anspruch 7, **dadurch gekennzeichnet, dass** die täglich verabreichte Menge an ASS im Bereich von ca. 40 bis ca. 60 mg liegt, vorzugsweise ca. 50 mg beträgt.

9. Acetylsalicylsäure (ASS) zur Verwendung nach Anspruch 7 oder 8, **dadurch gekennzeichnet, dass** die Verabreichung des Medikamentes zumindest 1 Tag, vorzugsweise 5 bis 7 Tage vor Einsatz der Membranlunge beginnt.

10. Acetylsalicylsäure (ASS) zur Verwendung nach einem der Ansprüche 7 bis 9, **dadurch gekennzeichnet, dass** die Verabreichung des Medikamentes zumindest 1 Tag, vorzugsweise 5 bis 7 Tage vor Ende des Einsatzes der Membranlunge beendet wird.

11. Acetylsalicylsäure (ASS) zur Verwendung nach einem der Ansprüche 7 bis 10, **dadurch gekennzeichnet, dass** die Verabreichung des Medikamentes im Zusammenhang mit einer Behandlung mit einem extrakorporalen oder implantierbaren Lungenunterstützungssystem erfolgt.

12. Acetylsalicylsäure (ASS) zur Verwendung nach einem der Ansprüche 7 bis 11, **dadurch gekennzeichnet, dass** die Verabreichung des Medikamentes oral oder intravenös in einer zu der oral verabreichten Menge äquivalenten Dosis erfolgt.

## Claims

1. Use of acetylsalicylic acid (ASA) in the preparation of a medicament which is administered to a human patient in connection with a treatment of the patient with a membrane lung, in order to avoid or reduce a deposition of blood constituents in the membrane lung, **characterised in that** the administration of the medicament involves ASA initially being administered once, several times or continuously each day in a therapeutically effective daily amount that lies below 500 mg, preferably below 100 mg, and aspirin (ASA) is used as the sole therapeutically active substance in the preparation of the medicament.

2. The use according to claim 1, **characterized in that** the daily administered amount of ASA is in the range of about 40 to about 60 mg, preferably is about 50 mg.

3. The use according to claim 1 or 2, **characterized in that** the administration of the medicament begins at least 1 day, preferably 5 to 7 days before application of the membrane lung.

4. The use according to any of claims 1 to 3, **characterized in that** the administration of the medicament is terminated at least 1 day, preferably 5 to 7 days before the end of the application of the membrane lung.

5. The use according to any of claims 1 to 4, **characterized in that** the administration of the medicament takes place in connection with a treatment with an extracorporeal or implantable lung assist system.

6. The use according to any of claims 1 to 5, **characterized in that** the medicament is administered orally, or is administered intravenously at a dose equivalent to the orally administered amount.

7. Acetylsalicylic acid (ASA) for use in connection with a treatment of a human patient with a membrane lung, in order to avoid or reduce a deposition of blood constituents in the membrane lung, **characterised in that** the ASA is administered as medicament to the patient and the administration of the medicament involves ASA initially being administered once several times or continuously each day in a therapeutically effective daily amount that lies below 500 mg, preferably below 100 mg, and aspirin (ASA) is used as the sole therapeutically active substance.

8. Acetylsalicylic acid (ASA) for use according to claim 7, **characterized in that** the daily administered amount of ASA is in the range of about 40 to about 60 mg, preferably is about 50 mg.

9. Acetylsalicylic acid (ASA) for use according to claim 7 or 8, **characterized in that** the administration of the medicament begins at least 1 day, preferably 5 to 7 days before application of the membrane lung.

10. Acetylsalicylic acid (ASA) for use according to any of claims 7 to 9, **characterized in that** the administration of the medicament is terminated at least 1 day, preferably 5 to 7 days before the end of the application of the membrane lung.

11. Acetylsalicylic acid (ASA) for use according to any of claims 7 to 10, **characterized in that** the administration of the medicament takes place in connection with a treatment with an extracorporeal or implantable lung assist system.

12. Acetylsalicylic acid (ASA) for use according to any of claims 7 to 11, **characterized in that** the medicament is administered orally, or is administered intravenously at a dose equivalent to the orally administered amount.

## Revendications

1. Utilisation d'acide acétylsalicylique (AAS) dans la production d'un médicament qui est administré à un patient humain dans le contexte d'un traitement du patient avec un poumon à membrane pour éviter ou réduire un dépôt de composants sanguins dans le poumon à membrane, **caractérisée en ce que** l'administration du médicament comprend l'administration initiale de l'AAS une ou plusieurs fois par jour ou en continu d'une quantité quotidienne thérapeutiquement efficace qui est inférieure à 500 mg, de préférence, inférieure à 100 mg et **en ce que**, lors de la production du médicament, on utilise de l'aspirine (AAS) comme principe actif thérapeutique unique.

2. Utilisation selon la revendication 1, **caractérisée en ce que** la quantité d'AAS administrée quotidiennement est de l'ordre d'environ 40 à environ 60 mg, de préférence, est d'environ 50 mg.

3. Utilisation selon la revendication 1 ou 2, **caractérisée en ce que** l'administration du médicament commence au moins 1 jour, de préférence 5 à 7 jours avant l'utilisation du poumon à membrane.

4. Utilisation selon l'une des revendications 1 à 3, **caractérisée en ce que** l'administration du médicament se termine au moins 1 jour, de préférence 5 à 7 jours avant la fin de l'utilisation du poumon à membrane.

5. Utilisation selon l'une des revendications 1 à 4, **caractérisée en ce que** l'administration du médicament s'effectue en relation avec un traitement avec un système de soutien pulmonaire extracorporel ou implantable.

6. Utilisation selon l'une des revendications 1 à 5, **caractérisée en ce que** l'administration du médicament s'effectue par voie orale ou intraveineuse en une dose équivalente à la quantité administrée par voie orale.

7. L'acide acétylsalicylique (AAS) pour utilisation dans le contexte d'un traitement du patient humain avec un poumon à membrane pour éviter ou réduire un dépôt de composants sanguins dans le poumon à membrane, **caractérisé en ce que** l'AAS est administré comme le médicament au patient et l'administration du médicament comprend l'administration initiale de l'AAS une ou plusieurs fois par jour ou en continu d'une quantité quotidienne thérapeutiquement efficace qui est inférieure à 500 mg, de préférence, inférieure à 100 mg et **en ce que** on utilise de l'aspirine (AAS) comme principe actif thérapeutique unique.

8. L'acide acétylsalicylique (AAS) pour utilisation selon la revendication 7, **caractérisé en ce que** la quantité d'AAS administrée quotidiennement est de l'ordre d'environ 40 à environ 60 mg, de préférence, est d'environ 50 mg.

9. L'acide acétylsalicylique (AAS) pour utilisation selon la revendication 7 ou 8, **caractérisé en ce que** l'administration du médicament commence au moins 1 jour, de préférence 5 à 7 jours avant l'utilisation du poumon à membrane.

10. L'acide acétylsalicylique (AAS) pour utilisation selon l'une des revendications 7 à 9, **caractérisé en ce que** l'administration du médicament se termine au moins 1 jour, de préférence 5 à 7 jours avant la fin de l'utilisation du poumon à membrane.

11. L'acide acétylsalicylique (AAS) pour utilisation selon l'une des revendications 7 à 10, **caractérisé en ce que** l'administration du médicament s'effectue en relation avec un traitement avec un système de soutien pulmonaire extracorporel ou implantable.

12. L'acide acétylsalicylique (AAS) pour utilisation selon l'une des revendications 7 à 11, **caractérisé en ce que** l'administration du médicament s'effectue par voie orale ou intraveineuse en une dose équivalente à la quantité administrée par voie orale.
